# EUROPEAN PATENT SPECIFICATION

(11) **EP 2 573 169 B1**
(45) Date of publication and mention of the grant of the patent: **26.10.2016**
(21) Application number: 10851841.6
(22) Date of filing: 08.12.2010
(51) Int. Cl.: C12N 5/10, C12N 15/85, C12N 5/073, A01K 67/027, C07K 14/47

(54) **CARTILAGE-SPECIFIC HIF-2 TRANSGENIC MOUSE AS AN ANIMAL MODEL FOR ARTHRITIS, AND USE THEREOF**
KNORPELSPEZIFISCHE HIF-2 TRANSGENE MAUS ALS TIERMODELL FÜR ARTHRITIS UND ANWENDUNG DAVON
SOURIS TRANSGÉNIQUE HIF-2 SPÉCIFIQUE DU CARTILAGE COMME MODÈLE ANIMAL POUR L'ARTHRITE, ET SON UTILISATION

(30) Priority: 20.05.2010 KR 20100047592
(43) Date of publication of application: 27.03.2013
(73) Proprietor: Gwangju Institute of Science and Technology, Gwangju 500-712 (KR)
(72) Inventor: CHUN, Jang-Soo, Gwangju 500-712 (KR); YANG, Si Young, Gwangju 502-260 (KR)
(74) Representative: Gevers & Orès
(86) International application number: PCT/KR2010/008736
(87) International publication number: WO 2011/145795

(56) References cited:
- JP-A- 2008 289 477
- KR-A- 20010 033 289
- US-A1- 2006 156 419
- YANG S ET AL: "216 REGULATION OF MATRIX METALLOPROTEINASES EXPRESSION BY EPAS1 IN ARTICULAR CHONDROCYTES", OSTEOARTHRITIS AND CARTILAGE, vol. 16, 1 September 2008 (2008-09-01), page S102, XP025689928, BAILLIERE TINDALL, LONDON, GB ISSN: 1063-4584, DOI: 10.1016/S1063-4584(08)60261-7 [retrieved on 2008-09-01]
- YANG SIYOUNG ET AL: "Hypoxia-inducible factor-2 alpha is a catabolic regulator of osteoarthritic cartilage destruction", NATURE MEDICINE, vol. 16, no. 6, June 2010 (2010-06), pages 687-693, XP002715738, ISSN: 1078-8956
- MUZ BARBARA ET AL: "Hypoxia. The role of hypoxia and HIF-dependent signalling events in rheumatoid arthritis.", ARTHRITIS RESEARCH & THERAPY 2009, vol. 11, no. 1, 201, 2009, XP002715739, ISSN: 1478-6362
- ELSON D A ET AL: "Induction of hypervascularity without leakage or inflammation in transgenic mice overexpressing hypoxia-inducible factor-1alpha", GENES AND DEVELOPMENT, vol. 15, no. 19, 1 October 2001 (2001-10-01), pages 2520-2532, XP002478666, COLD SPRING HARBOR LABORATORY PRESS, PLAINVIEW, NY, US ISSN: 0890-9369, DOI: 10.1101/GAD.914801
- KIM WILLIAM Y ET AL: "Failure to prolyl hydroxylate hypoxia-inducible factor alpha phenocopies VHL inactivation in vivo", EMBO (EUROPEAN MOLECULAR BIOLOGY ORGANIZATION) JOURNAL, vol. 25, no. 19, October 2006 (2006-10), pages 4650-4662, XP002715740, ISSN: 0261-4189
- UETA CHISATO ET AL: "Skeletal malformations caused by overexpression of cbfa1 or its dominant nagative form in chondrocyte", THE JOURNAL OF CELL BIOLOGY : JCB, vol. 153, no. 1, 1 April 2001 (2001-04-01) , pages 87-99, XP002957346, THE ROCKEFELLER UNIVERSITY PRESS, US ISSN: 0021-9525, DOI: 10.1083/JCB.153.1.87
- GIATROMANOLAKI, A. ET AL.: 'Upregulated hypoxia inducible factor-lalpha and -2alpha pathway in rheumatoid arthritis and osteoarthritis' ARTHRITIS RESEARCH & THERAPY vol. 5, no. 4, 29 April 2003, pages R193 - R201, XP021020700
- TALKS K L ET AL: "The expression and distribution of the hypoxia-inducible factors HIF-1alpha and HIF-2alpha in normal human tissues, cancers, and tumor-associated macrophages", AMERICAN JOURNAL OF PATHOLOGY; [10640], ELSEVIER INC, US, vol. 157, no. 2, 1 August 2000 (2000-08-01), pages 411-421, XP002223397, ISSN: 0002-9440
- BLANCHER CHRISTINE ET AL: "Relationship of hypoxia-inducible factor (HIF)-1alpha and HIF-2alpha expression to vascular endothelial growth factor induction and hypoxia survival in human breast cancer cell lines", CANCER RESEARCH, vol. 60, no. 24, 15 December 2000 (2000-12-15), pages 7106-7113, ISSN: 0008-5472

<110> GIST
<120> Transgenic Mice Overexpressing HIF-2a as an Animal Model of Arthritis and Uses Thereof
<130> PP100105
<150> KR 10-2010-0047592
   <151> 2010-05-20
<160> 7
<170> KopatentIn 1.71
<210> 1
   <211> 5352
   <212> DNA
   <213> Mus musculus
<400> 1
   ttttttttct ttttttcttt ttcttttttt ctttttcttt tttggttggt tggttttgat      60
   ttgtcagatc ccagaaaagt gactcctgtt cggggctaaa cggaactcca ggtcccttgt     120
   cgctgctctc tctctctttt ggcgtcttac aacctcctcc cactcctttc cccggccccg     180
   cctcctcctg caggttcctc cccgtcaccc ccctcctccc tcctcctcct ccgcacctag     240
   ccagccctct gcaaacttcc acctgattga gcgggactct cggacctgcg agcactaaag     300
   acctttcaca cctgcccggg cgacagagag ctgcggaggg ccacagcaaa gagagcggct     360
   gcagccccta cggggttaag gaacccaggt gctccgggtc tcggagggcc acggcgacaa     420
   tgacagctga caaggagaaa aaaaggagca gctcagagct gaggaaggag aaatcccgtg     480
   atgccgcgag gtgccggcgc agcaaggaga cggaggtctt ctatgagttg gctcatgagt     540
   tgcccctgcc tcacagtgtg agctcccacc tggacaaagc ctccatcatg cgcctggcca     600
   tcagcttcct tcggacacat aagctcctgt cctcagtctg ctctgaaaat gaatctgaag     660
   ctgaggccga ccagcaaatg gataacttgt acctgaaagc cttggagggt ttcattgctg     720
   tggtgaccca agacggtgac atgatctttc tgtcggaaaa catcagcaag ttcatgggac     780
   ttactcaggt agaactaaca ggacacagca tctttgactt cactcatcct tgcgaccatg     840
   aggagatccg tgagaacctg actctcaaaa acggctctgg ttttgggaag aagagcaaag     900
   acgtgtccac cgagcgtgac ttcttcatga ggatgaagtg cacggtcacc aacagaggcc     960
   ggactgtcaa cctcaagtcg gccacctgga aggtcctgca ctgcaccggg caagtgagag    1020
   tctacaacaa ctgcccccct cacagtagcc tctgtggctc caaggagccc ctgctgtcct    1080
   gccttatcat catgtgtgag ccaatccagc acccatccca catggacatc cccctggaca    1140
   gcaagacttt cctgagccgc cacagcatgg acatgaagtt cacctactgt gacgacagaa    1200
   tcttggaact gattggttac caccccgagg agctacttgg acgctctgcc tatgagttct    1260
   accatgccct ggattcggag aacatgacca aaagtcacca gaacttgtgc accaaggggc    1320
   aggtggtatc tggccagtac cggatgctag ccaaacacgg aggatatgtg tggctggaga    1380
   cccaggggac ggtcatctac aacccccgca acctgcagcc tcagtgtatc atgtgtgtca 1440
   actatgtgct gagtgagatc gagaagaacg acgtggtgtt ctccatggac cagaccgaat 1500
   ccctgttcaa gccacacctg atggccatga acagcatctt tgacagcagt gacgatgtgg 1560
   ctgtaactga gaagagcaac tacctgttca ccaaactgaa ggaggagccc gaggaactgg 1620
   cccagttggc ccccacccca ggagatgcca ttatttctct cgatttcgga agccagaact 1680
   tcgatgaacc ctcagcctat ggcaaggcca tccttccccc gggccagcca tgggtctcgg 1740
   ggctgaggag ccacagtgcc cagagcgagt ccgggagcct gccagccttc actgtgcccc 1800
   aggcagacac cccagggaac actacaccca gtgcttcaag cagcagtagc tgctccacgc 1860
   ccagcagccc tgaggactac tattcatcct tggagaatcc cttgaagatc gaagtgattg 1920
   agaagctttt cgccatggac acggagccga gggacccggg cagtacccag acggacttca 1980
   gtgaactgga tttggagacc ttggcaccct acatccctat ggacggcgag gacttccagc 2040
   tgagccccat ctgcccagag gagccgctca tgccagagag cccccagccc accccccagc 2100
   actgcttcag taccatgacc agcatcttcc agccgctcac cccgggggcc acccacggcc 2160
   ccttcttcct cgataagtac ccgcagcagt tggaaagcag gaagacagag tctgagcact 2220
   ggcccatgtc ttccatcttc tttgatgctg ggagcaaagg gtccctgtct ccatgctgtg 2280
   gccaggccag cacccctctc tcttctatgg gaggcagatc caacacgcag tggcccccgg 2340
   atccaccatt acatttcggc cctactaagt ggcctgtggg tgatcagagt gctgaatccc 2400
   tgggagccct gccggtgggg tcatcgcagt tggaacctcc gagcgccccg cctcatgtct 2460
   ccatgttcaa gatgaggtct gcaaaggact tcggggcccg aggtccatac atgatgagcc 2520
   cagccatgat cgccctgtcc aacaagctga agctaaagcg gcagctggag tatgaggagc 2580
   aagccttcca agacacaagc gggggggacc ctccaggcac cagcagttca cacttgatgt 2640
   ggaaacgtat gaagagcctc atgggcggga cctgtccttt gatgcctgac aagaccatca 2700
   gtgcgaacat ggcccccgat gaattcaccc aaaaatctat gagaggcctg ggccagccac 2760
   tgagacacct gccacctccc cagccaccat ctaccaggag ctcaggggag aacgccaaga 2820
   ctgggttccc gccacagtgc tatgcctccc agttccagga ctacggtcct ccaggagctc 2880
   aaaaggtgtc aggcgtggcc agtcgactgc tggggccatc gttcgagcct tacctgttgc 2940
   cggaactgac cagatatgac tgtgaggtga acgtgcccgt gcctggaagc tccacactcc 3000
   tgcaggggag agaccttctc agagctctgg accaggccac ctgagccagg gcctctggcc 3060
   gggcatgccc ctgcctgccc cgccgtcttg acctgccagc ttcacttcca tctgtgttgc 3120
   tattaggtat ctctaacacc agcacacttc ttacgagatg tactcaacct ggcctactgg 3180
   ccaggtcacc aagcagtggc ctttatctga catgctcact ttattatcca tgttttaaaa 3240
   atacatagtt gttgtacctg ctatgtttta ccgttgatga aagtgttctg aaattttata 3300
   agatttcccc ctccctccct cccttgaatt acttctaatt tatattcccc aaaggttttt 3360
   ctctctctca ttcatatcca tactaacaag catggtggct ggtgcctctc cctaggaaag 3420
   ctttggcgtc attcaactca agtgttcttg ttcttgttgc caaagagaaa aggattttcc 3480
   tccactgtgg attctccctc tcccccaccc ccacatacac acacacacac acacacccct 3540
   acacacatat acacacatgc acgtatgcgt gcacacacac acacacacac acacacacac 3600
   acacacaccc ctacacacac acacacacac acacatatac acacacacac acacacacac 3660
   acacacccct acacacatat acacacatgc acgtatgcgt gcacacacac acacacacac 3720
   acacacaccc ctacacacat atacacacat gcacgtatgc gtgcacacac acacacacac 3780
   acacacatct aatcaccata ttgtaaaatt ttgtgttttt aaagccaact ctttgctccg 3840
   gttttttcat acgacttagt atggggcaaa aaagcaatgt gaagaatcaa ctctagggtt 3900
   acctgtgaag ccacgcggtg gtgttcgaag ctgtctggta atgcccccat ctctccccgg 3960
   gtccagtgga tttttttaac tattattcaa aagcaaaact gagttttgtt ttgtttggtt 4020
   ttttaagaag aatttatatc cgggtttagt gtttatcata tatatgggta ctctgtaata 4080
   tctaaaagct tagaaatggg atcttgctca caagaatcac ttttaagatc ttttagggct 4140
   gttaatttta tttttctcag tgttctggac actgtagacc tgtgcagtac tcccacaggc 4200
   ctgactaacc gccacagatc tctctttctt tctctctggt gactttttgc ttttgtgata 4260
   tggcagcggt gtgacagtcc caggagagaa gtcattaagg gggaacattg caagctatct 4320
   tttcgtgttc tcagtgtatt atgtacgtgt gtgctgttgc cgctatgagg gctcggggct 4380
   tgcaggctct gagggcatgt cgcaggcgag ctggaaggaa gcgctctcac tttttttcag 4440
   gcaatgttgc tagcccttct agcacactga gctacgagac tgccaatctc tctcctgcgg 4500
   cacgtttggg ttggcacagc taccatgaaa tgtttttaat gggaattcat aaagcaagtt 4560
   aggattcaca aatgtagtga cctagtcctt ggagctaaac tgggagtggg tgccaggtgg 4620
   agggtagcag gtctgaccag ccctacaccc tgccacagat cacagcttgc cgggcaaatg 4680
   accctgcgaa gccacggtgc agcgccccct gctggcgagc agcgggcaga cctccaagga 4740
   cagcctctag ctgaggtctc agtcacatga agtgtgacac tgaacaggta gaaagcactg 4800
   aaaatcgtgt tccaagagca ctttgtaact cactgagggg gaggggaagc accttttggg 4860
   tttgcgatac caatcaacat aaggtgttgt tgactcgggt acaataaaac tctcaacaca 4920
   cacaaagccc atcaggcaag agtacagtaa gcctaagggg acttgctgct caaaccaaaa 4980
   attaactctg aaggtcagtc tacctgacat tacctaggca tggtgttgtc caagctgcgc 5040
   atcggtgcac agtgtggcct ggcacacacg caggcgggcg acggaacttg aagggttatt 5100
   gacatgcaaa tgctggtgtt tgatttcctg tgttgttgcc tcagcgttaa gggcatttcc 5160
   gtttgcagtt ctactaaaga cactctgaga aatattccga gtttcgtatt aacctttcct 5220
   gtccatgtaa caacttcatg accattactg cattgtcaaa ttcctactga cgacattata 5280
   actgtacggg agcttaactt tataaggaaa tgtattttga cactgatatc ttattaaagt 5340
   attctgatcc ta 5352
<210> 2
   <211> 20
   <212> DNA
   <213> Artificial Sequence
<220>
   <223> HIF-2a sense-primer
<400> 2
   gcctcatgtc tccatgttca 20
<210> 3
   <211> 21
   <212> DNA
   <213> Artificial Sequence
<220>
   <223> HIF-2a antisense-primer
<400> 3
   tgtatcttat catgtctgca t 21
<210> 4
   <211> 22
   <212> DNA
   <213> Artificial Sequence
<220>
   <223> Mouse HIF-2a sense-primer
<400> 4
   cgagaagaac gacgtggtgt tc 22
<210> 5
   <211> 19
   <212> DNA
   <213> Artificial Sequence
<220>
   <223> Mouse HIF-2a antisense-primer
<400> 5
   gtgaaggcgg gcaggctcc 19
<210> 6
   <211> 19
   <212> DNA
   <213> Artificial Sequence
<220>
   <223> Mouse GAPDH sense-primer
<400> 6
   tcactgccac ccagaagac 19
<210> 7
   <211> 19
   <212> DNA
   <213> Artificial Sequence
<220>
   <223> Mouse GAPDH antisense-primer
<400> 7
   tgtaggccat gaggtccac 19

## Description

### FIELD OF THE INVENTION

The present invention relates to a cartilage-specific HIF-2α transgenic mouse as an animal model for arthritis, and use thereof.

### DESCRIPTION OF THE RELATED ART

Degenerative arthritis (osteoarthritis, OA) is a representative degenerative disease that is primarily characterized by gradual and irreversible articular cartilage destruction and it leads to degrade the quality of life. Peoples aged more than 70 years old represent a degenerative arthritis and approximately 1/4 of them require clinical treatment. The market size of arthritis continues to increase is approximately 9.5 billion dollars in the world, growing by an annual average 14% and expected to approximately 20 billion dollars in 2005 and approximately 350 billion dollars in 2010. Currently, it now has an estimated 400 billion won in domestic market. However, no effective medical therapy to prevent OA cartilage destruction is currently available, owing to the limited understanding of the underlying molecular pathogenic mechanisms. A variety of potential OA-causing mechanisms have been proposed.

Pathological causes of degenerative arthritis have been known to be induced by biochemical changes in the articular cartilage which is caused by the mechanical damage of the joints, genetic factors and obesity. This in turn leads to activation of biochemical pathways in chondrocytes, a unique resident cell type that synthesizes cartilagespecific extracellular matrix (ECM) components as well as various catabolic and anabolic factors. Activation of biochemical pathways involves the production of proinflammatory cytokines, inflammation, degradation of the ECM by matrix metalloproteinases (MMPs) and ADAMTS, and cessation of ECM synthesis via dedifferentiation and apoptosis of chondrocytes. However, the molecular pathogenic mechanisms of osteoarthritis need to be elucidated in more detail.

Meanwhile, the HIF-2α (also known as endothelial PAS domain protein-1 or EPAS1) is gene regulated via hypoxea as transcription factor and it induces expression of target genes to allow adopting hypoxea. However, function of HIF-2α in cartilage degeneration is not yet clearly understood, except for a report that HIF-2α is expressed in hypertrophic chondrocyte.

### DETAILED DESCRIPTION OF THIS INVENTION

### Technical Purposes of This Invention

The present inventors have made intensive studies to develop novel animal model system for a more efficient research to arthritis. As a result, they have introduced recombinant expression vector overexpressing hypoxia-inducible factor 2α (HIF-2 α) to a fertilized egg of mouse (Deposit accession number: KTCT11701BP), constructed a transgenic mouse, and found out that the transgenic mouse may be very useful as an animal model system for preventing or treating arthritis.

Accordingly, it is an object of this invention to provide a transgenic animal model for arthritis obtained from a non-human transgenic fertilized egg.

It is another object of this invention to provide a method for screening a therapeutic agent for treating arthritis.

Other objects and advantages of the present invention will become apparent from the detailed description to follow taken in conjugation with the appended claims and drawings.

### DETAILED DESCRIPTION OF THIS INVENTION

In one aspect of the present invention, there is provided a transgenic animal model for arthritis obtained from a non-human transgenic fertilized egg comprising an expression construct comprising (a) a nucleotide sequence encoding HIF-2α (hypoxia-inducible factor 2α); and (b) a transcription-regulating sequence operatively linked to the nucleotide, wherein the transcription-regulating sequence comprises a type II collagenase promoter, a type II collagenase enhancer, and an intron sequence between the promoter and the nucleotide sequence encoding HIF-2α, wherein the transgenic fertilized egg has been deposited at the Korea Research Institute of Bioscience and Biotechnology (KRIBB) on May 20, 2010 under accession number KCTC 11701BP, wherein the HIF-2α in the transgenic animal for arthritis increases the expression of cartilage degeneration-inducing factor.

The present inventors have made intensive studies to develop novel animal model system for a more efficient research to arthritis. As a result, they have introduced recombinant expression vector overexpressing hypoxia-inducible factor 2α (HIF-2 α) to a fertilized egg of mouse (Deposit accession number: KTCT11701BP), constructed a transgenic mouse, and found out that the transgenic mouse may be very useful as an animal model system for preventing or treating arthritis.

Arthritis means diseases of joint disorder that involves inflammation caused by various factors. Specially, osteoarthritis (OA) is one of the oldest and the most common disease. It means a chronic state characterized by destruction of joint's cartilage and is known as degenerative joint disease, ostoarthrosis, hypertrophic arthritis or degenerative arthritis. Therefore, the term used herein "osteoarthritis" and other names above-mentioned will be used interchangeably. Cartilage is one area of the joint that it serves as cushion at tail end of bones to support moving joint easily. Cartilage destruction induces an abrasion between the adjacent bones and lead to stiffness and suffering caused by difficulty of the movement of the joints.

Arthritis is very high incidence disease which is estimated approximately 200 million people in Korea and approximately 2,700 million in the United States (Helmick, C., et al of the Prevalence of Arthritis and OtherRheumatic conditions. Arthritis & Rheumatism, 58 (1): 15-25 (2008)). Unfortunately, a clear understanding for cause of the disease is scarce yet so that there is no therapeutic method. Actually, therapeutic method can be a variety of ways depending on the situation, because arthritis is caused by various factors (for example, age, obesity, injury, excessive use of joint and genetic cause).

The present invention provides a transgenic animal, preferably a mouse, and a animal model system for arthritis using recombinant expression vector (expression construct) comprising HIF-2α as cartilage degeneration-inducing factor and its controllable target factor.

The recombinant expression vector of the present invention comprises the nucleotide sequence as set forth in SEQ ID NO:1. More detailed, the recombinant expression vector comprises (a) a construct comprising an expression substance of interest-encoding nucleotide sequence; (b) a promoter which is operatively linked to the construct and act in animal cells to form RNA molecule, more preferably (a) a construct comprising a nucleotide sequence encoding hypoxia-inducible factor 2α (HIF-2 α) as set forth in SEQ ID NO:1; (b) a transcription-regulating sequence which is operatively linked to the construct, still more preferably (a) a construct comprising a nucleotide sequence encoding hypoxia-inducible factor 2α (HIF-2 α) as set forth in SEQ ID NO:1; (b) a transcription-regulating sequence which is operatively linked to the construct; and (c) a poly A signal causing polyadenylation at the 3'-end of a RNA molecule which act in animal cells, most preferably (a) a construct comprising a nucleotide sequence encoding CII-pNass-β-HIF-2α; (b) a transcription-regulating sequence which is operatively linked to the construct; and (c) a poly A signal causing polyadenylation at the 3'-end of a RNA molecule which act in animal cells.

The mRNA level or the protein level of the expression of the nucleotide sequence as set forth in SEQ ID NO:1 which is included in the recombinant vector of the present invention is increased in arthritis induced cells (*e*.*g*., chondrocytes) or tissues (*e*.*g*., the transgenic mouse cartilage tissue in the present invention) so that researches associated with arthritis have usability in animals (preferably mouse). In addition, it may become apparent to those skilled in this art that variants of nucleotide sequence encoding HIF-2α belong to the present invention. In other words, it may become apparent to those skilled in this art that variants fused with marker for detecting target protein belong to the present invention. For example, the protein which may be fused to target protein for detecting target protein includes, but not limited to, GFP (green fluorescent protein), RFP (red fluorescent protein), CFP (cyan fluorescent protein) and YFP (yellow fluorescent protein), BFP (bluefluorescent protein), luciferase or its variants (*e.g*., EGFP, ECFP, EYFP, ERFP, EBFP).

According to a preferable embodiment, the HIF-2α in transgenic animal model for arthritis of the present invention increases the expression of cartilage degeneration-inducing factor. According to a preferred embodiment, the cartilage degeneration-inducing factor of the present invention includes, but not limited to, matrix metalloprotenase (MMP) -1, MMP-3, MMP-9, MMP-12, MMP-13, ADAMTS (a disintegrin and metalloproteinase with thrombospondin motifs)-4, ADAMTS-5, PTGS-2 (Prostaglandin-endoperoxide synthase-2) or NOS2 (nitric oxide synthase 2).

The term used herein "transcription-regulating sequence" means a DNA sequence that regulates the expression of a coding sequence or a functional RNA. The term "operatively linked" refers to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence.

According to a preferable embodiment, the transcription-regulating sequence comprises a promoter and an enhancer.

The transcription-regulating sequence linked to the HIF-2α is operable in, preferably animal cells, more preferably mammalian cells, still more preferably human or mouse cells, most preferably chondrocytes to control transcription of the HIF-2α, including the promoters derived from the genome of mammalian cells or from mammalian viruses., Examples of such promoters are CMV (cytomegalovirus) promoter, CII promoter, the adenovirus late promoter, the vaccinia virus 7.5K promoter, SV40 promoter, HSV tk promoter, RSV promoter, EF1 alpha promoter, metallothionein promoter, beta-actin promoter, human IL-2 gene promoter, human IFN gene promoter, human IL-4 gene promoter, human lymphotoxin gene promoter, human GM-CSF gene promoter, inducible promoter, tumor cell specific promoter (*e.g.*, TERT promoter, PSA promoter, PSMA promoter, CEA promoter, E2F promoter and AFP promoter) and tissue specific promoter (*e.g*., albumin promoter).

In the present invention, the promoter is a chondrocyte-specific promoter, the CII promoter, and more preferably, the present invention further comprise the enhancer, most preferably the chondrocyte-specific enhancer of type II collagenase.

According to a preferable embodiment, the expression construct of the present invention further comprises an intron sequence between the promoter and the nucleotide sequence encoding HIF-2α.

Preferably, the expression constructs used in the present invention comprises polyadenylated sequence (*e.g*., bovine growth hormone terminator and polyadenylated sequence derived from SV40).

According to a preferred embodiment, the construct comprising HIF-2α-encoding nucleotide sequence used in the present invention has the structure of "transcription-regulating sequence- HIF-2α-encoding nucleotide sequence-polyadenylated sequence".

The HIF-2α-encoding nucleotide sequence of the present invention comprises any of gene delivery system used in gene therapy by those skilled in the art, preferably, plasmid, adenovirus (Lockett LJ, et al., Clin. Cancer Res., 3:2075-2080(1997)), adeno-associated virus (AAV, Lashford LS., et al., Gene Therapy Technologies, Applications and Regulations Ed. A. Meager, 1999), retrovirus (Gunzburg WH, et al., Retroviral vectors. Gene Therapy Technologies, Applications and Regulations Ed. A. Meager, 1999), lentivirus (Wang G. et al., J. Clin. Invest. 104(11):R55-62(1999)), herpes simplex virus (Chamber R., et al., Proc. Natl. Acad. Sci USA, 92:1411-1415(1995)), vaccinia virus (Puhlmann M. et al., Human Gene Therapy, 10:649-657(1999)) liposome ((Methods in Molecular Biology, Vol 199, S.C. Basu and M. Basu (Eds.), Human Press 2002)) or niosome. Most preferably, the gene delivery system of this invention is constructed by incorporating the HIF-2α-encoding nucleotide sequence to adenovirus.

### i. Adenovirus

Adenoviruses have been usually employed as a gene delivery system because of its mid-sized genome, ease of manipulation, high titer, wide target-cell range, and high infectivity. Both ends of the viral genome contains 100-200 bp ITRs (inverted terminal repeats), which are cis elements necessary for viral DNA replication and packaging. The E1 region (E1A and E1B) encodes proteins responsible for the regulation of transcription of the viral genome and a few cellular genes. The expression of the E2 region (E2A and E2B) results in the synthesis of the proteins for viral DNA replication.

Of adenoviral vectors developed so far, the replication incompetent adenovirus having the deleted E1 region is usually used. The deleted E3 region in adenoviral vectors may provide an insertion site for transgenes (Thimmappaya, B. et al., Cell, 31:543-551(1982); and Riordan, J. R. et al., Science, 245:1066-1073(1989)). Therefore, it is preferred that the nucleotide sequence encoding the CCN5 protein or CCN2ΔCT protein of this invention is inserted into either the deleted E1 region (E1A region and/or E1B region, preferably, E1B region) or the deleted E3 region, more preferably, the deleted E3 region. The nucleotide sequence of interest to be delivered is preferably inserted into either the deleted E1 region (E1A region and/or E1B region, preferably, E1B region) or the deleted E3 region, more preferably, the deleted E1 region. Furthermore, the inserted sequences may be incorporated into the deleted E4 region. The term used herein "deletion" with reference to viral genome encompasses whole deletion and partial deletion as well.

According to the most preferred embodiment of the present invention, the adenovirus gene transduction system of the present invention comprises "promoter-the HIF-2α gene-poly A sequence". The "promoter-the HIF-2α gene-poly A sequence"is inserted into the deleted E1 region (E1A region and/or E1B region, preferably, E1B region) or the E3 region, preferably, the deleted E3 region.

In nature, adenovirus can package approximately 105% of the wild-type genome, providing capacity for about 2 extra kb of DNA (Ghosh-Choudhury et al., EMBO J., 6:1733-1739(1987)). In this regard, the foreign sequences described above inserted into adenovirus may be further inserted into adenoviral wild-type genome.

The adenovirus may be of any of the 42 different known serotypes or subgroups A-F. Adenovirus type 5 of subgroup C is the most preferred starting material for constructing the adenoviral gene delivery system of this invention. A great deal of biochemical and genetic information about adenovirus type 5 is known.

The foreign genes delivered by the present adenoviral gene delivery system are episomal, and therefore, have low genotoxicity to host cells. Therefore, gene therapy using the adenoviral gene delivery system of this invention may be considerably safe.

### ii. Retrovirus

Retroviruses capable of carrying relatively large exogenous genes have been used as viral gene delivery vectors in the senses that they integrate their genome into a host genome and have broad host spectrum.

In order to construct a retroviral vector, the HIF-2α gene is inserted into the viral genome in the place of certain viral sequences to produce a replication-defective virus. To produce virions, a packaging cell line containing the gag, pol and env genes but without the LTR (long terminal repeat) and Ψ components is constructed (Mann et al., Cell, 33:153-159(1983)). When a recombinant plasmid containing the HIF-2α gene, LTR and Ψ is introduced into this cell line, the Ψ sequence allows the RNA transcript of the recombinant plasmid to be packaged into viral particles, which are then secreted into the culture media (Nicolas and Rubinstein "Retroviral vectors," In: Vectors: A survey of molecular cloning vectors and their uses, Rodriguez and Denhardt (eds.), Stoneham: Butterworth, 494-513(1988)). The media containing the recombinant retroviruses is then collected, optionally concentrated and used for gene delivery.

A successful gene transfer using the second-generation retroviral vector has been reported. Kasahara et al. (Science, 266:1373-1376(1994)) prepared variants of moloney murine leukemia virus in which the EPO (erythropoietin) sequence is inserted in the place of the envelope region, consequently, producing chimeric proteins having novel binding properties. Likely, the present gene delivery system can be constructed in accordance with the construction strategies for the second-generation retroviral vector.

### iii. AAV vector

Adeno-associated viruses are capable of infecting non-dividing cells and various types of cells, making them useful in constructing the gene delivery system of this invention. The detailed descriptions for use and preparation of AAV vector are found in U.S. Pat. No. 5,139,941 and 4,797,368.

Research results for AAV as gene delivery systems are disclosed in LaFace et al, Viology, 162:483486(1988), Zhou et al., Exp. Hematol. (NY), 21:928-933(1993), Walsh et al, J. Clin. Invest., 94:1440-1448(1994) and Flotte et al., Gene Therapy, 2:29-37(1995). Recently, an AAV vector has been approved for Phase I human trials for the treatment of cystic fibrosis.

Typically, a recombinant AAV virus is made by cotransfecting a plasmid containing the gene of interest (i.e., relaxin gene and nucleotide sequence of interest to be delivered) flanked by the two AAV terminal repeats (McLaughlin et al., 1988; Samulski et al., 1989) and an expression plasmid containing the wild type AAV coding sequences without the terminal repeats (McCarty et al., J. Virol., 65:2936-2945(1991)).

### iv. Other viral vectors

Other virus vectors may be used for the gene transduction system in the present invention. Vectors derived from viruses such as vaccinia virus (Puhlmann M. et al., Human Gene Therapy 10:649-657(1999); Ridgeway, "Mammalian expression vectors," In: Vectors: A survey of molecular cloning vectors and their uses. Rodriguez and Denhardt, eds. Stoneham: Butterworth, 467-492(1988); Baichwal and Sugden, "Vectors for gene transfer derived from animal DNA viruses: Transient and stable expression of transferred genes," In: Kucherlapati R, ed. Gene transfer. New York: Plenum Press, 117-148(1986) and Coupar et al., Gene, 68:1-10(1988)), lentivirus (Wang G. et al., J. Clin. Invest. 104(11):R55-62(1999)) and herpes simplex virus (Chamber R., et al., Proc. Natl. Acad. Sci USA 92:1411-1415(1995)) may be used in the present delivery systems for transferring the HIF-2α gene into cells.

### v. Liposome

Liposomes are formed spontaneously when phospholipids are suspended in an excess of aqueous medium. Liposome-mediated nucleic acid delivery has been very successful as described in Nicolau and Sene, Biochim. Biophys. Acta, 721:185-190(1982) and Nicolau et al., Methods Enzymol., 149:157-176(1987). Example of commercially accessible reagents for transfecting animal cells using liposomes includes Lipofectamine (Gibco BRL). Liposomes entrapping the HIF-2α gene interact with cells by mechanism such as endocytosis, adsorption and fusion and then transfer the sequences into cells.

The methods Introduced gene delivery system of the present invention into cells the above-mentioned may be carried out through a variety of methods known in the art. Where the present gene delivery system is constructed on the basis of viral vector construction, the contacting is performed as conventional infection methods known in the art. The infection of hosts using viral vectors is well described in the above-cited publications. Where the present gene delivery system is a naked recombinant DNA molecule or plasmid, the nucleotide sequence to be delivered are introduced into cells by microinjection (Capecchi, M.R., Cell, 22:479(1980) and Harland and Weintraub, J. Cell Biol. 101:1094-1099(1985)), calcium phosphate co-precipitation (Graham, F.L. et al., Virology, 52:456(1973) and Chen and Okayama, Mol. Cell. Biol. 7:2745-2752(1987)), electroporation (Neumann, E. et al., EMBO J., 1:841(1982) and Tur-Kaspa et al., Mol. Cell Biol., 6:716-718(1986)), liposome-mediated transfection (Wong, T.K. et al., Gene, 10:87(1980) and Nicolau and Sene, Biochim. Biophys. Acta, 721:185-190(1982); and Nicolau et al., Methods Enzymol., 149:157-176(1987)), DEAE-dextran treatment (Gopal, Mol. Cell Biol., 5:1188-1190(1985)) and particle bombardment (Yang et al., Proc. Natl. Acad. Sci., 87:9568-9572(1990)), and most preferably microinjection.

According to the present invention, the recombinant expression vector of the present invention is microinjected into the fertilized egg to obtain the transgenic fertilized egg. The fertilized egg with the gene inserted was implanted in the womb of a surrogate (for example, mouse) to obtain mice. Then, 10 transgenic mice were selected through PCR genotyping.

PCR is one of the most predominant processes for nucleic acid amplification and a number of its variations and applications have been developed. For example, for improving PCR specificity or sensitivity, touchdown PCR, hot start PCR, nested PCR and booster PCR have been developed with modifying traditional PCR procedures. In addition, real-time PCR, differential display PCR (DD-PCR), rapid amplification of cDNA ends (RACE), multiplex PCR, inverse polymerase chain reaction (IPCR), vectorette PCR and thermal asymmetric interlaced PCR (TAIL-PCR) have been suggested for certain applications. The details of PCR can be found in McPherson, M.J., and Moller, S.G. PCR. BIOS Scientific Publishers, Springer-Verlag New York Berlin Heidelberg, N.Y. (2000).

### SUBSTITUTE SHEET

The term used herein "amplification" refers to reactions for amplifying nucleic acid molecules. A multitude of amplification reactions have been suggested in the art, including polymerase chain reaction (hereinafter referred to as PCR) (U.S. Patent Nos. 4,683,195, 4,683,202, and 4,800,159), reverse transcription-polymerase chain reaction (hereinafter referred to as RT-PCR) (Sambrook, J. et al., Molecular Cloning. A Laboratory Manual, 3rd ed. Cold Spring Harbor Press (2001)), the methods of Miller, H. I. (WO 89/06700) and Davey, C. et al. (EP 329,822), ligase chain reaction (LCR), Gap-LCR (WO 90/01069), repair chain reaction (EP 439,182), transcription-mediated amplification (TMA; WO 88/10315), self sustained sequence replication (WO 90/06995), selective amplification of target polynucleotide sequences (U.S. Patent No. 6,410,276), consensus sequence primed polymerase chain reaction (CP-PCR; U.S. Patent No. 4,437,975), arbitrarily primed polymerase chain reaction (AP-PCR; U.S. Patent Nos. 5,413,909 and 5,861,245), nucleic acid sequence based amplification (NASBA; U.S. Patent Nos. 5,130,238, 5,409,818, 5,554,517 and 6,063,603), strand displacement amplification and loop-mediated isothermal amplification (LAMP), but not limited to. Other amplification methods that may be used are described in U.S. Patent Nos. 5,242,794, 5,494,810, 4,988,617 and in U.S. Ser. No. 09/854,317.

Therefore, the present invention may be generally carried out by nucleic acid amplifications using mRNA molecules in samples as templates and primers to be annealed to mRNA or cDNA.

For obtaining mRNA molecules, total RNA is isolated from samples. The isolation of total RNA may be performed by various methods (Sambrook, J. et al., Molecular Cloning. A Laboratory Manual, 3rd ed. Cold Spring Harbor Press (2001); Tesniere, C. et al., Plant Mol. Biol. Rep., 9: 242 (1991); Ausubel, F.M. et al., Current Protocols in Molecular Biology, John Willey & Sons (1987); and Chomczynski, P. et al., Anal. Biochem. 162: 156 (1987)). For example, total RNA in cells may be isolated using Trizol. Afterwards, cDNA molecules are synthesized using mRNA molecules isolated and then amplified. Since total RNA molecules used in the present invention are isolated from human samples, mRNA molecules have poly-A tails and converted to cDNA by use of dT primer and reverse transcriptase *(*PNAS USA, 85: 8998 (1988); Libert F, etal., Science, 244: 569 (1989); and Sambrook, J. et al., Molecular Cloning. A Laboratory Manual, 3rd ed. Cold Spring Harbor Press (2001)). cDNA molecules synthesized are then amplified by amplification reactions.

The primers used for the present invention are hybridized or annealed to a region on template so that double-stranded structure is formed. Conditions of nucleic acid hybridization suitable for forming such double stranded structures are described by Joseph Sambrook, et al., Molecular Cloning, A Laboratory Manual, Cold Spring Harbor Laboratory Press, Cold Spring Harbor, N.Y. (2001) and Haymes, B. D., et al., Nucleic Acid Hybridization, A Practical Approach, IRL Press, Washington, D.C. (1985).

A variety of DNA polymerases can be used in the amplification step of the present methods, which includes "Klenow" fragment of *E. coli* DNA polymerase I, a thermostable DNA polymerase and bacteriophage T7 DNA polymerase. Preferably, the polymerase is a thermostable DNA polymerase obtained from a variety of bacterial species, including *Thermus aquaticus* (Taq), *Thermus thermophilus* (Tth), *Thermus filiformis, Thermis flavus, Thermococcus literalis,* and *Pyrococcus furiosus* (Pfu).

When a polymerization reaction is being conducted, it is preferable to provide the components required for such reaction in excess in the reaction vessel. Excess in reference to components of the amplification reaction refers to an amount of each component such that the ability to achieve the desired amplification is not substantially limited by the concentration of that component. It is desirable to provide to the reaction mixture an amount of required cofactors such as Mg²⁺, and dATP, dCTP, dGTP and dTTP in sufficient quantity to support the degree of amplification desired. All of the enzymes used in this amplification reaction may be active under the same reaction conditions. Indeed, buffers exist in which all enzymes are near their optimal reaction conditions. Therefore, the amplification process of the present invention can be done in a single reaction volume without any change of conditions such as addition of reactants.

Annealing or hybridization in the present invention is performed under stringent conditions that allow for specific binding between the target nucleotide sequence and the primer. Such stringent conditions for annealing will be sequence-dependent and varied depending on environmental parameters.

The composition of the present invention may optionally include other reagents along with components described above. For instance, where the present kit may be used for nucleic acid amplification, it may optionally include the reagents required for performing PCR reactions such as buffers, DNA polymerase (thermostable DNA polymerase obtained from *Thermus aquaticus* (Taq), *Thermus thermophilus* (Tth), *Thermus filiformis, Thermis flavus*, *Thermococcus literalis,* and *Pyrococcus furiosus* (Pfu)), DNA polymerase cofactors, and dNTPs.

Primers used in the the present invention have a sequence complementary to a nucleotide sequence encoding HIF-2α. The term "complementary" with reference to sequence used herein refers to a sequence having complementarity to the extent that the sequence hybridizes or anneals specifically with the nucleotide sequence described above under certain hybridization or annealing conditions. In this regard, the term "complementary" used herein has different meaning from the term "perfectly complementary". The primer of this invention may include one or more mismatch base sequences where it is able to specifically hybridize with the above-described nucleotide sequences.

The term "primer" used herein means a single-stranded oligonucleotide which is capable of acting as a point of initiation of template-directed DNA synthesis when placed under proper conditions (*i.e.,* in the presence of four different nucleoside triphosphates and a thermostable enzyme) in an appropriate buffer and at a suitable temperature. The suitable length of primers will depend on many factors, including temperature, application and source of primer, generally, 15-30 nucleotides in length. In general, shorter primers need lower temperature to form stable hybridization duplexes to templates.

The sequences of primers are not required to have perfectly complementary sequence to templates. The sequences of primers may comprise some mismatches, so long as they can be hybridized with templates and serve as primers. Therefore, the primers of this invention are not required to have perfectly complementary sequence to the nucleotide sequence as described above; it is sufficient that they have complementarity to the extent that they anneals specifically to the nucleotide sequence of the gene for acting as a point of initiation of synthesis. The primer design may be conveniently performed with referring to the above-described nucleotide sequences. For instance, the primer design may be carried out using computer programs for primer design (*e.g*., PRIMER 3 program).

To prepare primers, the nucleotide sequence of the present biomarker may be found in the GenBank Accession No. NM_010137.3 of HIF-2α (SEQ ID NO:1) and the GenBank Accession No. NM_008084.2 of GAPDH, and primers may be designed by reference with the nucleotide sequence afore-mentioned.

In another aspect of the present invention, there is provided a method for screening a therapeutic agent for treating arthritis, which comprises the step of analyzing expression levels of HIF-2α or the cartilage tissue destruction level of the transgenic animal which has been contacted by a test substance of interest for analysis, wherein where the test substance inhibits the expression of HIF-2α, or alleviates or improves the cartilage tissue destruction, it is determined as therapeutic agent for treating arthritis.

Since the method of the present invention follows the process of analyzing the expression levels of HIF-2α of the present method described above, the common descriptions between them are omitted in order to avoid undue redundancy leading to the complexity of this specification.

In the method for screening of the present invention, test substance or expression substance of interest-encoding nucleotide sequence or protein may be labeled by detectable label. For example, the detectable label is chemical label (*e.g.,* biotin), enzyme (horseradish peroxidase, alkaline phosphatase, peroxidase, luciferase, β-galactosidase, β-glucosidase), radioactive label (*e.g*., C¹⁴, I¹²⁵, P³² and S³⁵), fluorescent label [*e.g*., coumarin, fluorescein, FITC (fluoresein Isothiocyanate), rhodamine 6G, rhodamine B, TAMRA (6-carboxy-tetramethyl-rhodamine), Cy-3, Cy-5, Texas Red, Alexa Fluor, DAPI (4,6-diamidino-2-phenylindole), HEX, TET, Dabsyl and FAM], luminescent label, chemiluminescent label, FRET (fluorescence resonance energy transfer) label or metal label (*e.g.,* gold and silver)

According to the method of the present invention, a transgenic animal is prepared first using the recombinant expression vector of the present invention. The transgenic animal, preferably mouse is contacted to a test substance to be analyzed. The transgenic animal of the present invention is transgenic which is introduced a recombinant expression vector overexpressing HIF-2α. The term "test substance" used herein in conjunction with the present screening method refers to a material tested in the present method for analyzing the influence on the expression level of HIF-2α. The test substance includes siRNA (small interference RNA), shRNA (short hairpin RNA), miRNA (microRNA), ribozyme, DNAzyme, PNA (peptide nucleic acids), antisense oligonucleotides, antibodies, aptamers, extracts of natural sources and chemical substances, but not limited to.

Afterwards, the expression of the HIF-2α coding nucleotide sequence or the binding affinity of the transcription-regulating sequence to the HIF-2α protein in cells treated with the test substance is measured. The measurement of the expression levels may be carried out according to methods as described above, and the test substance may be determined as the therapeutic agent for treating arthritis where the test substance inhibits the expression of the HIF-2α protein or inhibits the binding of the transcription-regulating sequence to the HIF-2α.

The measurement of change in the expression level of the nucleotide sequence afore-mentioned may be carried out through a variety of methods known in the art, for example, RT-PCR (Sambrook et al, Molecular Cloning. A Laboratory Manual, 3rd ed. Cold Spring Harbor Press(2001)), Northern blot (Peter B. Kaufma et al., Molecular and Cellular Methods in Biology and Medicine, 102-108, CRC press), hybridization using cDNA microarray (Sambrook et al, Molecular Cloning. A Laboratory Manual, 3rd ed. Cold Spring Harbor Press(2001)) or *in situ* hybridization (Sambrook et al, Molecular Cloning. A Laboratory Manual, 3rd ed. Cold Spring Harbor Press (2001)).

According to a preferred embodiment, the present invention may be performed to the manner of immunoassay, *i*.*e*., antigen-antibody reaction manner. At this time, it is performed using an antibody or an aptamer specific binding to arthritis marker (*e.g.,* HIF-2α) of the present invention mentioned-above.

The antibody used in the present invention is polyclonal or monoclonal antibody, preferably monoclonal antibody. Antibody production may be prepared by a method widely known in the art, such as a hybridoma method (Kohler and Milstein, European Journal of Immunology, 6:511-519(1976)), a recombinant DNA methods (U.S. Patent No. 4,816,56) or a phage antibody library technique (Clackson et al, Nature, 352:624-628(1991) and Marks et al, J. Mol. Biol., 222:58, 1-597(1991)). General process for antibody production is described in Harlow, E. and Lane, D., Using Antibodies: A Laboratory Manual, Cold Spring Harbor Press, New York, 1999; Zola, H., Monoclonal Antibodies: A Manual of Techniques, CRC Press, Inc., Boca Raton, Florida, 1984; Coligan, CURRENT PROTOCOLS IN IMMUNOLOGY, Wiley / Greene, NY, 1991. For example, the preparation of the hybridoma cells producing monoclonal antibody is accomplished by fusing immortalized cell line with antibody-producing lymphocytes. The technology required for this process is widely known in the art and may be easily carried out using techniques. Polyclonal antibodies may be produced by injecting the protein antigen into an appropriate animal and collecting blood samples from the animal to obtain sera containing antibodies using affinity technology known in the art.

When the method of the present disclosure is performed using an antibody or an aptamer, the present disclosure may be used for diagnosing arthritis by performing conventional immunoassay methods.

The immunoassay may be performed by a variety of quantitative or qualitative immunoassay protocols. The immunoassay format includes, but is not limited to, radioimmunoassay analysis, radioactive immunoprecipitation, immunoprecipitation, immunohistochemical staining, ELISA (enzyme-linked immunosorbant assay), Capture-ELISA, sandwich assay, flow cytometry, immunofluorescence and immune affinity purified. The immunoassay or the immuno staining method is described in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984; and Ed Harlow and David Lane, Using Antibodies:A Laboratory Manual, Cold Spring Harbor Laboratory Press, 1999.

For example, when the method of the present disclosure is performed using radioimmunoassay analysis, the antibody labled with radioactive isotopes (*e.g*., C¹⁴, I¹²⁵, P³² and S₃₅) may be used for detecting a marker of the present invention.

When the method of the present disclosure is performed using the ELISA, certain examples of the present invention comprise the steps of: (i) coating unknown cell cytolysate samples of interest for analysis on the surface of a solid substrate; (ii) contacting the cell cytolysate with antibody for the marker as the primary antibody; (iii) contacting the resultant of step (ii) with the secondary antibody conjugated enzyme; and (iv) detecting the enzyme activity.

The appropriate solid substrate is hydrocarbon polymers (*e.g*., polystyrene and polypropylene), glass, metal, or gel, and most preferably a micro-titer plate.

The appropriate secondary antibody conjugated enzyme includes, but is not limited to, color-developing reaction, fluorescent reaction, luminescent reaction or infrared reaction, for example, alkaline phosphatase, β-galactosidase, horseradish peroxidase, luciferase and cytochrome P450. Where alkaline phosphatase is used for the enzyme binding to the secondary antibody, bromochloroindolylphosphate (BCIP), nitro blue tetrazolium (NBT), naphthol-AS-B1-phosphate and ECF (enhanced chemifluorescence) may be used as a substrate for color-developing reactions. In the case of using horseradish peroxidase, chloronaphtol, aminoethylcarbazol, diaminobenzidine, D-luciferin, lucigenin (bis-N-methylacridinium nitrate), resorufin benzyl ether, iuminol, Amplex Red reagent (10-acetyl-3,7-dihydroxyphenoxazine), HYR (p-phenylenediamine-HCl and pyrocatechol), TMB (3,3,5,5-tetramethylbenzidine), ABTS (2,2-Azine-di[3-ethylbenzthiazoline sulfonate]), o-phenylenediamine (OPD) and naphtol/pyronine may be used as a substrate; and in the case of using glucose oxidase, t-NBT (nitroblue tetrazolium) or m-PMS (phenzaine methosulfate) may be used as a substrate.

When the method of the present disclosure is performed using the Capture-ELISA, certain examples of the present invention comprise the steps of: (i) coating the antibody for the marker as capturing antibody on the surface of a solid substrate; (ii) contacting the sample with the capturing antibody; (iii) contacting the resultant of step (ii) with the detecting antibody which is combined with label generating signal and react specifically to the HIF-2α protein; and (iv) detecting the signal from the label.

The detecting antibody has the label generating detectable a signal. The label includes, but is not limited to, chemical (*e.g.,* biotin), enzyme (alkaline phosphatase, β-galactosidase, horseradish peroxidase and cytochrome P450), radioactive material (*e.g.,* C¹⁴, I¹²⁵, P³² and S³⁵), fluorescent material (*e.g.,* fluorescein), luminescent material, chemiluminescent material and FRET (fluorescence resonance energy transfer). A variety of labels and labeling methods are described in Ed. Harlow and David Lane, Using Antibodies: A Laboratory Manual, Cold Spring Harbor Laboratory Press, 1999.

The final measurement of enzyme activity or measurement of the signal may be carried out in accordance with a variety of methods known in the art. The detection of this signal permits to qualitative or quantitative analysis of the marker of the present invention. In the case of using biotin as label, the signal is easily detected using streptavidin. In the case of using luciferase as label, the signal is easily detected using luciferin.

According to another embodiment of this invention, the present invention may use aptamers specific to markers instead of antibodies. Aptamers include oligo nucleic acid and peptide molecules, of which details can be found in Bock LC et al., Nature 355 (6360):5646 (1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24):142727 (1998).

### Advantageous Effects

The features and advantages of the present invention will be summarized as follows:
(a) The present invention relates to a transgenic animal model for arthritis obtained from a non-human transgenic fertilized egg comprising an expression construct comprising (a) a nucleotide sequence encoding HIF-2α (hypoxia-inducible factor 2α); and (b) a transcription-regulating sequence operatively linked to the nucleotide sequence.
(b) A transgenic mouse of the present invention may be very useful as a therapeutic model for arthritis, which may be used in studies on the cartilage degeneration process.
(c) In addition, the transgenic mouse of the present invention may be applied to a method for screening arthritis therapeutics.

### BRIEF DESCRIPTION OF THE DRAWINGS

Fig. 1 schematically represents the vector map required for preparing HIF-2α genetically engineered mouse.
Fig. 2 represents the result that vector DNA is inserted on chromosome of transgenic mouse and it is verified using PCR (polymerase chain reaction) and western blot.
Fig. 3 represents the immunohistochemical analysis of the cartilage destruction level in HIF-2α transgenic mouse and wild-type mouse
Fig. 4 represents the result of RT-PCR (reverse transcription-polymerase chain reaction) and western blot analysis for cartilage degeneration inducing factors in cartilage of HIF-2α transgenic mouse and wild-type mouse.

### SUBSTITUTE SHEET

### EXAMPLES OF THE INVENTION

The present invention will now be described in further detail by examples. It would be obvious to those skilled in the art that these examples are intended to be more concretely illustrative and the scope of the present invention as set forth in the appended claims is not limited to or by the examples.

### EXAMPLES

### Example 1: Vector preparation for manufacture of chondrocyte-specific HIF-2α transgenic mouse and production of transgenic mouse

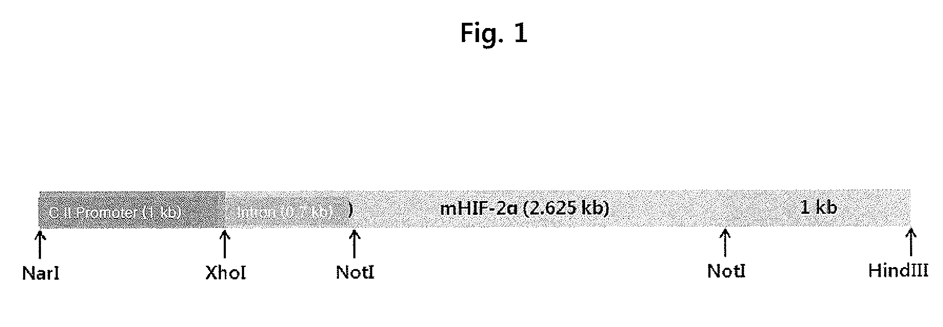

To prepare the transgenic mouse overexpressing chondrocyte-specific HIF-2α, a gene for transplantation was prepared first (Fig. 1).

A fragment (2.625 kb) of a mouse HIF-2α gene cleaved by *NotI* and *NotI* restriction enzymes was cloned into the *NotI* site of 4.35-kb pNass-beta vector (Clontech Laboratories, USA). In addition, enhancer, promoter and 0.7 kb intron parts of 1.0 kb type II collagen (Col2a1) inducing chondrocyte-specific expression were cloned into the *NotI* site of the vector. The vector was cleaved with *NotI* to obtain a purified, 9.675-kb gene for transplantation. The gene is called a CII-pNass-β-HIF-2α construct (Fig. 1; gene 2.625 kb + vector 4.35 kb + enhancer 1.0 kb + promoter 1.0 kb + intron 0.7 kb = 9.675 kb). The gene for transplantation was microinjected into the pronucleus of a fertilized egg of a C57BL/6 mouse (Charles River, Japan). The fertilized egg obtained as a resultant was deposited to Korea Research Institute of Bioscience and Biotechnology at May 20, 2010 and received a deposit accession number KTCT11701BP. The fertilized egg with the gene inserted was implanted in the womb of a surrogate mouse to obtain at least 200 mice. Then, 10 transgenic mice were selected through PCR genotyping.

### Example 2: Gene analysis of chondrocyte-specific HIF-2a transgenic mouse

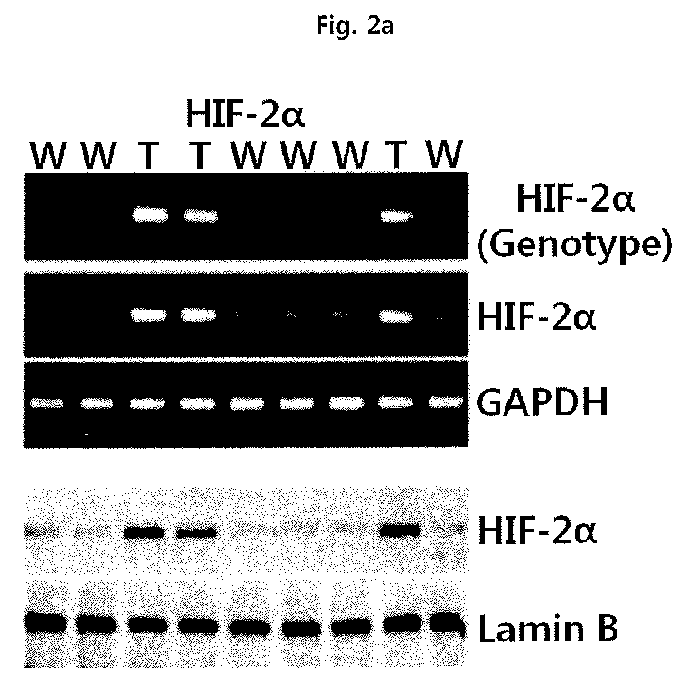

The original mouse obtained in the Example 1 was mated with wild-type mouse. The resultant mouse genomic DNA was extracted from tail of the mouse and subject to PCR gene analysis using primers in which may specifically amplify only cDNA of HIF-2α transgenic mouse (Fig. 2a).

The Primers used in PCR are described in Table 1 and PCR reaction condition is as follows: the reaction mixture was denatured for 5 min at 95°C, subjected to 30 cycles of 10 sec at 95°C, 20 sec at 58°C and 30 sec at 72°C and extended for 10 min at 72 °C.

**[Table 1]**

| Primer sequence | | | | | |
|---|---|---|---|---|---|
| Gene | | Primer sequence | SEQ ID NO | Predicted size | Use |
| HIF-2α gene test | sense | 5'-GCCTCATGTCTCCATGTTCA-3' | 2 | 628 bp | HIF-2α TG gene test |
| | antisense | 5'-TGTATCTTATCATGTCTGCAT-3' | 3 | | |

### Example 3: Gene expression verification of chondrocyte-specific HIF-2α transgenic mouse

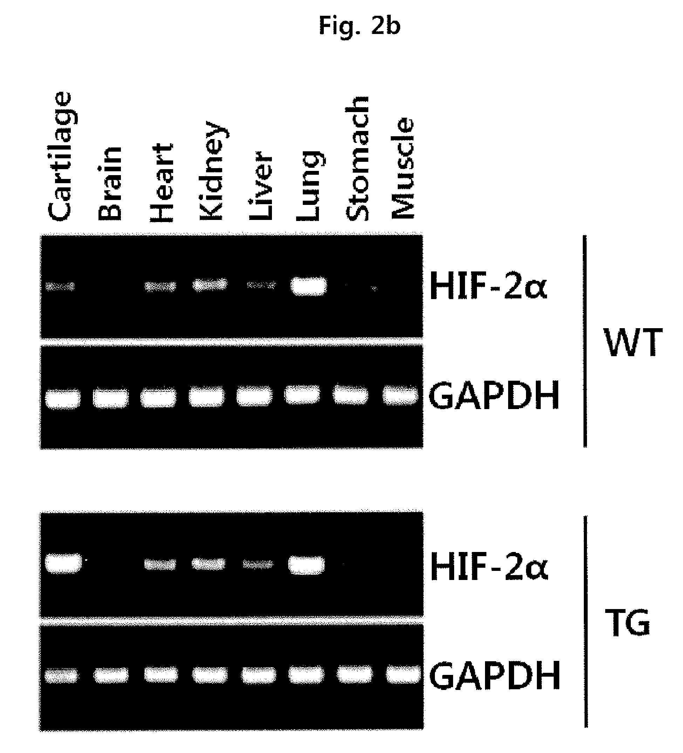

We examined whether HIF-2α is overexpressed in HIF-2α transgenic mouse tissues (cartilage, brain, heart, kidney, liver, lung, stomach, muscle) and chondrocytes using western blot and PCR. As a result, it was demonstrated that HIF-2α was overexpressed specifically in only cartilage tissue (Fig. 2a and 2b).

### 3-1. Western blot and PCR

To perform western blot extracting protein in cells, the mouse knee joint chondrocytes were isolated from the knee joint of 8-day-old mouse and cultured in DMEM (Gibco, USA) containing 10% (v/v) fetal bovine serum (Gibco, USA), 50 µg/mL streptomycin (Sigma-Aldrich, USA) and 50 unit/mL penicillin (Sigma-Aldrich, USA). A monoclonal mouse anti-HIF-2α antibody (Abcam., Inc. USA) was used.

In addition, RNAs were extracted from chondrocytes and tissues (cartilage, brain, heart, kidney, liver, lung, stomach, muscle) in wild-type mouse and HIF-2α transgenic mouse, and subjected to PCR to verify mRNA expression levels. Total RNA used in RT-PCR was isolated from respective tissues and cells using RNA STAT-60 (Tel-Test, Woodlands, TX) and reverse transcription was performed using ImProm-IITM reverse transcriptase (Promega, Madison, WI). The produced cDNA was amplified by PCR using Taq polymerase (TaKaRa Bio, Shiga, Japan). Primers used in PCR are described in Table 2.

**[Table 2]**

| Primer sequence | | | | |
|---|---|---|---|---|
| Gene | | Primer sequence | SEQ ID NO | Predicted size |
| Mouse HIF-2α | sense | 5'-CGAGAAGAACGACGTGGTGTTC-3' | 4 | 293 bp |
| | antisense | 5'-GTGAAGGCGGGCAGGCTCC-3 | 5 | |
| Mouse GAPDH | sense | 5'-TCACTGCCACCCAGAAGAC-3' | 6 | 450 bp |
| | antisense | 5'-TGTAGGCCATGAGGTCCAC-3 | 7 | |

As a result, it would be demonstrated that the protein and mRNA of HIF-2α were overexpressed strongly in HIF-2α transgenic mouse chondrocytes, in contrast, the expression of HIF-2α was expressed weakly in wild-type mouse. In the results of mRNA expression levels of HIF-2α for each of tissues, mRNA expression of HIF-2α was significantly increased in only HIF-2α transgenic mouse cartilage, on the other hand, mRNA expression of HIF-2α was expressed weakly in wild-type mouse. Taken together, it would be understood that HIF-2α was overexpressed in only transgenic mouse chondrocytes which is prepared by the method of the present invention.

### Example 4: Phenotype investigation of HIF-2α transgenic mouse

### 4-1. Immunohistochemistry

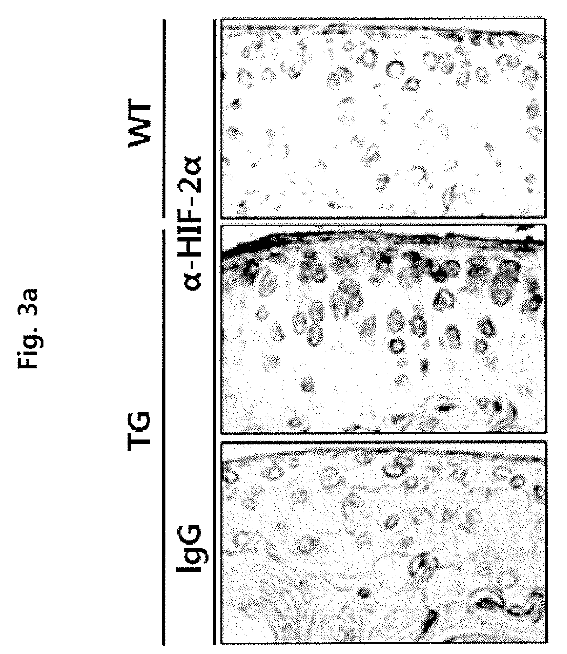

HIF-2α protein expression levels in transgenic mouse cartilage tissue were verified using immunohistochemistry. The cartilage of 45-week-old transgenic mouse or wild-type mouse was fixed in 4% paraformaldehyde (PFA, Sigma-Aldrich, USA) solution for 24 hours, incubated in 10% EDTA (Sigma-Aldrich, USA) solution for 2 weeks and prepared into a paraffin (McCormick, USA) block by increasing alcohol concentration. The paraffin block was sliced to 6-µm thickness, deparaffinised in xylene (Sigma-Aldrich, USA) and treated with 10%(v/v) H₂O₂ (Sigma-Aldrich, USA) in PBS (Sigma-Aldrich, USA) to remove activity of peroxidase in tissue. The slides were blocked with 1% BSA (Sigma-Aldrich, USA) and incubated at 4°C with a monoclonal mouse anti-HIF-2α antibody. Normal mouse IgG was used as negative control (Fig. 3a).

As a result, it would be demonstrated that the protein of HIF-2α was overexpressed strongly in HIF-2α transgenic mouse cartilage tissue, in contrast, there was no HIF-2α expression in wild-type mouse cartilage tissue. These facts mean that HIF-2α protein was expressed in only transgenic mouse because transgenic mouse prepared to allow high-expressing HIF-2α in cartilage tissue which is abundant type II collagen (FIG. 3a).

### 4-2. Histological observation

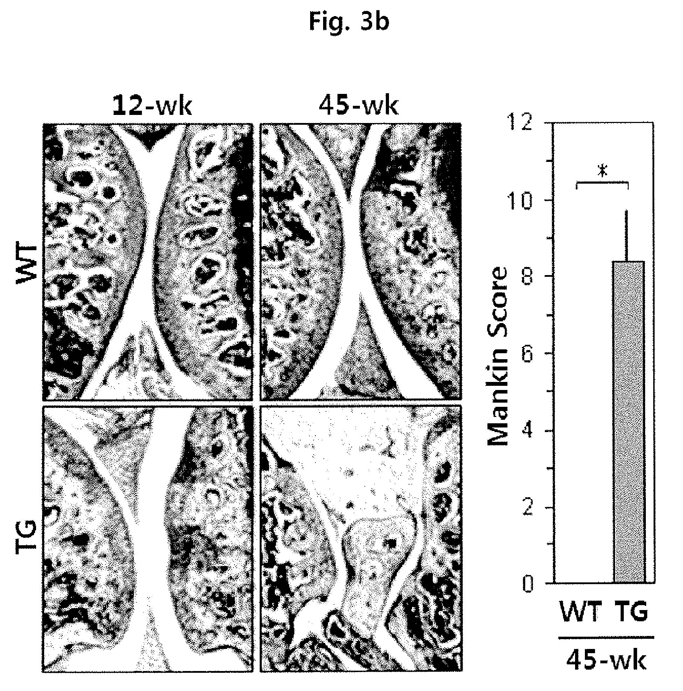

In order to examine cartilage destruction level in the transgenic mice, the cartilage of 12-week-old and 45-week-old transgenic mouse or wild-type mouse was fixed in 4% paraformaldehyde (PFA, Sigma-Aldrich, USA) solution for 24 hours, incubated in 10% EDTA solution for 2 weeks and prepared into a paraffin block by increasing alcohol concentration. The paraffin block was sliced to 6-µm thickness and the slides were stained with safranin-O (Sigma-Aldrich, USA) and hematoxylin (Sigma-Aldrich, USA) (Fig. 3b).

As a result, when compared with the 12-week-old wild-type mouse, slight irregularity was found in the HIF-2α transgenic mouse. In addition, when compared with the 45-week-old wild-type mouse, the cartilage of the HIF-2α transgenic mouse showed thinner and damaged cartilage tissue. The Mankin score indicative of cartilage damage was remarkably higher in the HIF-2α transgenic mouse than in the wild-type mouse, indicating that degenerative arthritis was induced (Fig. 3b).

### 4-2. PCR

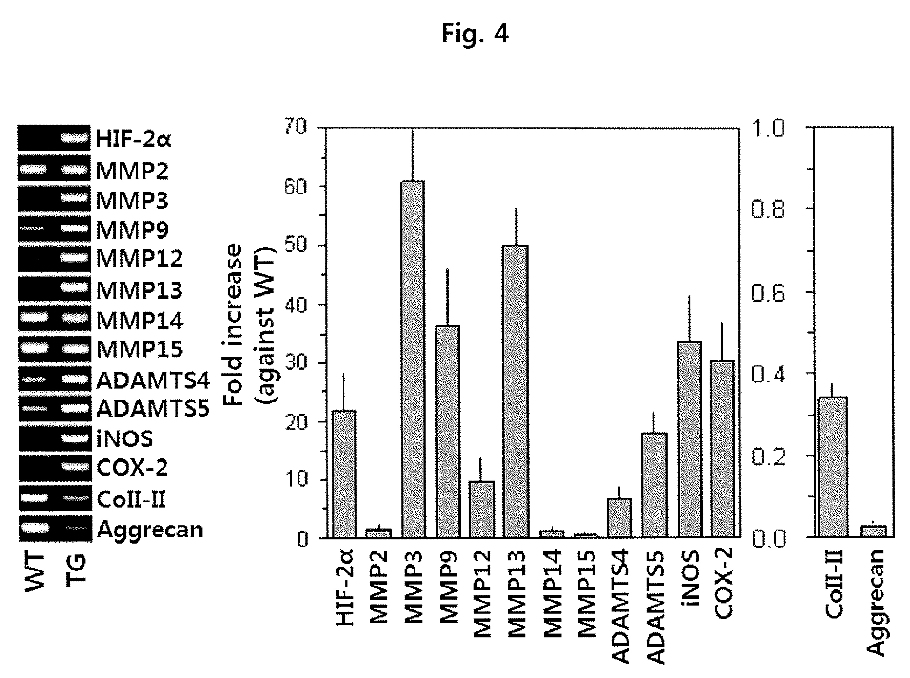

In order to examine the expression level of cartilage degeneration-inducing factor, RNAs were extracted from cartilages in 45-week-old transgenic mouse or wild-type mouse and the expression of representative cartilage degeneration-inducing factors such as MMP, ADAMTS, COX-2 and iNOS, were verified using RT-PCR (Fig. 4).

As a result, HIF-2α was overexpressed in HIF-2α transgenic mouse cartilage tissue than in the wild-type mouse, and mRNA expressions of MMP-3, MMP-9, MMP-12, MMP-13, ADAMTS-4, ADAMTS-5, COX-2 and iNOS were verified using RT-PCR and qRT-PCR (Fig. 4).
## Claims

1. A transgenic animal model for arthritis obtained from a non-human transgenic fertilized egg comprising an expression construct comprising (a) a nucleotide sequence encoding HIF-2α (hypoxia-inducible factor 2α); and (b) a transcription-regulating sequence operatively linked to the nucleotide,
wherein the transcription-regulating sequence comprises a type II collagenase promoter, a type II collagenase enhancer, and an intron sequence between the promoter and the nucleotide sequence encoding HIF-2α,
wherein the transgenic fertilized egg has been deposited at the Korea Research Institute of Bioscience and Biotechnology (KRIBB) on May 20, 2010 under accession number KCTC 11701BP,
wherein the HIF-2α in the transgenic animal for arthritis increases the expression of cartilage degeneration-inducing factor.

2. The transgenic animal model for arthritis according to claim 1, wherein the cartilage degeneration-inducing factor is matrix metalloprotenase (MMP)-3, MMP-9, MMP-12, MMP-13, ADAMTS (a disintegrin and metalloproteinase with thrombospondin motifs)-4, ADAMTS-5, COX-2 (Cyclooxygenase-2) or iNOS (inducible nitric oxide synthase).

3. The transgenic animal model for arthritis according to claim 1, wherein the animal is a mouse.

4. A method for screening a therapeutic agent for treating arthritis, which comprises the step of analyzing expression levels of HIF-2α or the cartilage tissue destruction level of a transgenic animal according to any of claims 1 and 2, which has been contacted with a test substance of interest for analysis,
wherein where the test substance inhibits the expression of HIF-2α, or alleviates or improves the cartilage tissue destruction, it is determined as therapeutic agent for treating arthritis.

5. The method according to claim 4, wherein the arthritis is osteoarthritis, degenerative arthritis, desquamative osteochondritis, meniscal injury, articular malalignment, avascular necrosis, rheumation arthritis, juvenile idiopathic arthritis, injury, inflammatory arthritis or septic arthritis caused by infection.

## Patentansprüche

1. Transgenes Tiermodell für Arthritis, das aus einem nichthumanen transgenen befruchteten Ovulum gewonnen ist und ein Expressionskonstrukt umfasst, das (a) eine für HIF-2α (Hypoxie-induzierenden Faktor 2α) codierende Nucleotidsequenz und (b) eine transkriptionsregulierende Sequenz umfasst, die mit dem Nucleotid funktionell gekoppelt ist,
wobei transkriptionregulierende Sequenz einen Promotor von Collagenase Typ II, einen Enhancer von Collagenase Typ II und eine Intron-Sequenz zwischen dem Promotor und der für HIF-2α codierenden Nucleotidsequenz umfasst,
wobei das transgene befruchtete Ovulum am 20. Mai 2010 beim Korea Research Institute of Bioscience and Biotechnology (KRIBB) unter der Zugangsnummer KCTC 11701BP hinterlegt wurde,
wobei der HIF-2α im transgenen Tier für Arthritis die Expression von Knorpeldegenerations-induzierendem Faktor verstärkt.

2. Transgenes Tiermodell für Arthritis nach Anspruch 1, wobei es sich bei dem Knorpeldegenerations-induzierenden Faktor um Matrixmetalloprotenase (MMP)-3, MMP-9, MMP-12, MMP-13, ADAMTS (ein Disintegrin und Metalloproteinase mit Thrombospondin-Motiven)-4, ADAMTS-5, COX-2 (Cyclooxygenase-2) oder iNOS (induzierbare Stickoxidsynthase) handelt.

3. Transgenes Tiermodell für Arthritis nach Anspruch 1, wobei das Tier eine Maus ist.

4. Verfahren zum Screening eines therapeutischen Mittels zur Behandlung von Arthritis, welches den Schritt des Analysierens von Expressionsstärken von HIF-2α oder des Knochengewebezerstörungsgrads eines transgenen Tieres nach einem der Ansprüche 1 und 2 umfasst, das mit einer Testsubstanz von Interesse für die Analyse in Kontakt gebracht wurde,
wobei die Testsubstanz, wenn sie die Expression von HIF-2α hemmt oder die Knochengewebezerstörung mindert oder verbessert, als therapeutisches Mittel zur Behandlung von Arthritis bestimmt wird.

5. Verfahren nach Anspruch 4, wobei es sich bei der Arthritis um Osteoarthritis, degenerative Arthritis, desquamative Osteochondritis, Meniskusverletzung, Gelenkfehlstellung, avaskuläre Nekrose, rheumatoide Arthritis, juvenile idiopathische Arthritis, Verletzung, entzündliche Arthritis oder septische Arthritis, die durch Infektion verursacht wird, handelt.

## Revendications

1. Animal transgénique comme modèle d'arthrite obtenu à partir d'un ovule transgénique fécondé non humain comprenant une construction d'expression comprenant (a) une séquence nucléotidique codant pour HIF-2α (facteur 2α inductible par hypoxie) ; et (b) une séquence de régulation de transcription liée fonctionnellement au nucléotide,
dans lequel la séquence de régulation de transcription comprend un promoteur de la collagénase de type II, un amplificateur de la collagénase de type II et une séquence intron entre le promoteur et la séquence nucléotidique codant pour HIF-2α,
dans lequel l'ovule transgénique fécondé a été déposé auprès du Korea Research Institute of Bioscience and Biotechnology (KRIBB) le 20 mai 2010 sous le numéro d'accès KCTC 117001BP,
dans lequel le HIF-2α dans l'animal transgénique modèle pour l'arthrite augmente l'expression du facteur d'induction de la dégénérescence cartilagineuse.

2. Animal transgénique comme modèle d'arthrite selon la revendication 1, dans lequel le facteur d'induction de la dégénérescence cartilagineuse est la métalloprotéinase matricielle (MMP)-3, MMP-9, MMP-12, MMP-13, ADAMTS-4 (une désintégrine et métalloprotéinase ayant des motifs de thrombospondine), ADAMTS-5, COX-2 (cyclo-oxygénase 2) ou iNOS (oxyde nitrique synthase inductible).

3. Animal transgénique comme modèle d'arthrite selon la revendication 1, l'animal étant une souris.

4. Procédé de détection d'un agent thérapeutique pour traiter l'arthrite, qui comprend l'étape consistant à analyser les niveaux d'expression de HIF-2α ou le niveau de destruction du tissu cartilagineux d'un animal transgénique selon l'une quelconque des revendications 1 et 2, qui a été mis en contact avec une substance à tester,
dans lequel lorsque la substance à tester inhibe l'expression de HIF-2α ou soulage ou réduit la destruction du tissu cartilagineux, elle est considérée comme étant un agent thérapeutique pour traiter l'arthrite.

5. Procédé selon la revendication 4, dans lequel l'arthrite est l'arthrose, l'arthrite dégénérative, l'ostéochondrite desquamative, une lésion du ménisque, un défaut d'alignement articulaire, une nécrose avasculaire, une arthrite rhumatoïde, une arthrite idiopathique juvénile, une lésion, une arthrite inflammatoire ou une arthrite septique provoquée par une infection.
